# EUROPEAN PATENT APPLICATION

(11) **EP 3 659 617 A1**
(43) Date of publication of application: **03.06.2020**
(21) Application number: 19210222.6
(22) Date of filing: 20.11.2019
(51) Int. Cl.: A61K 36/716, A61K 36/185, A61K 36/28, A61K 36/288, A61K 36/48, A61K 36/49, A61K 36/68, A61K 36/88, A61P 25/28, A23L 19/00, A23L 33/105

(54) **COMPOSITION FOR IMPROVING COGNITIVE FUNCTION INCLUDING CLEMATIS TERNIFLORA VAR. MANDSHURICA EXTRACT AS ACTIVE INGREDIENT**

(30) Priority: 27.11.2018 KR 20180148752
(71) Applicant: Famenity Co., Ltd., Gyeonggi-do (KR)
(72) Inventor: Lee, Ji Won, Gyeonggi-do (KR)
(74) Representative: Vossius & Partner Patentanwälte Rechtsanwälte mbB

(57) **Abstract**

The present invention relates to a composition for improving cognitive function, including a Clematis temiflora var. mandshurica extract as an active ingredient. The composition for improving cognitive function according to an exemplary embodiment of the present invention can provide a composition which is effective for stress relief, mood improvement, antidepressant and cognitive function improvement by utilizing an extract derived from natural products.

Further, the functional food composition of the present invention can be provided as a functional food composition with high preference while exhibiting effects of stress relief, antidepressant, and cognitive function improvement.

## Description

### [Technical Field]

The present invention relates to a composition for improving cognitive function, including a Clematis terniflora var. mandshurica extract as an active ingredient. More particularly, the present invention relates to a composition for improving cognitive function, which exhibits stress relief, mood improvement and antidepressant effects by including a Clematis terniflora var. mandshurica extract obtained from natural products as an active ingredient, and does not have any side effects due to long-term uptake of the composition.

### [Background Art]

Stress is a representative term relating to mental health and has long been a cause of all illnesses, and since stress excessively occurs regardless of gender and age due to various reasons such as social factors such as schoolwork, business, marriage, and childcare and surrounding environmental factors such as weather and traffic particularly in the modern society, stress has been recognized as a very important social problem. As the Korean society is rapidly developing and diversified, with an increase in the roles required for modern people, the number of people who complain of anxiety disorders and mental diseases caused by various stresses is increasing. Further, those who are older feel more stress, and the intensity of stress varies depending on the personality, hobby, stress-relieving method, surrounding environment, stress-controlling ability, and the like of an individual, but stress is mostly also accompanied by depression and fatigue.

Depression is a psychiatric disorder that may be caused by stress, also exhibits extreme results such as suicide, and has been recognized as a very important disease due to the high number of patient incidence along with high recurrence rate. The cause of depression has been revealed to be a disorder of brain neurotransmitters such as adrenaline, dopamine, or serotonin, and is also accompanied by brain damage such as hippocampal site atrophy and suppression of adult neurogenesis. A currently known representative therapeutic agent for depression is a tricyclic antidepressant (TCA), but has a disadvantage due to large side effects. In particular, amitriptyline and the like have been broadly prescribed in Korea, but have many problems such as various side effects.

Fatigue may sometimes be defined as "perception of reduced ability to physical and mental activity due to imbalances in the availability, utilization and recovery of the resources necessary to perform activities", and specifically, fatigue usually refers to a state in which the work ability is reduced due to physical fatigue, and stress usually refers to a state in which chaos of homeostasis occurs due to mental fatigue. When fatigue occurs continuously, fatigue may develop into a disease called "chronic fatigue syndrome" as a chronic condition. Currently, a sense of fatigue lasting for a long period of time as a main symptom of chronic fatigue syndrome (CFS) is a group of syndrome along with non-specific symptoms such as a slight fever, headache, sore throat, muscle joint pain, attention concentration disorder, memory loss, sleep disorder, and depression, and is generally free of obvious abnormalities in the case of physical and routine tests.

As described above, induction of anxiety, depression, and stress is closely related to in vivo neurotransmitters and hormones. In the body in response to external stimuli and stresses, the hypothalamus manages the secretion of neurotransmitters and hormones, but the hypothalamus regulates physiological activities such as emotional state, heart rate, blood pressure, and an increase in blood flow of skeletal muscle by regulating the release of neurotransmitters such as dopamine, noradrenaline, and serotonin at each nerve terminal through signaling to the central nervous system.

First, dopamine is a type of neurotransmitter in the form of catecholamine, serves to transmit the stimulation of cerebral nerve cells, and is known to mainly regulate the attention concentration, tension, and motivation state, and it is known that when dopamine is secreted excessively, a disease such as schizophrenia is caused, and when dopamine is deficient, movement disorders such as Parkinson's disease are caused.

Parkinson's disease (PD) is a type of neurodegenerative disease and was first reported as a degenerative cerebral nerve disease that causes motor and cognitive impairment by James Parkinson in 1817. The incidence of this disease is about 100 to 150 patients per 100,000 population in the United States, and about 750,000 to 1,000,000 patients have been reported, and generally 60,000 patients are newly diagnosed every year, and even in Korea, as the population ages, the incidence and prevalence thereof are expected to continuously increase. Histopathologically, the loss of dopamine neurons located in the substantia nigra characteristically occurs, the dopamine in the caudate nucleus and the putamen, which are the projection sites of nerve fibers, is reduced, and thus, motor and cognitive dysfunctions such as characteristic tremor, bradykinesia, rigidity, and disturbance of posture appear.

Examples of a main therapeutic drug used for Parkinson's disease include drugs that supplement the lack of dopamine function in the brain, treatment with drugs for regulating other purposes to prevent or delay the destruction of nerve cells, or other incidental symptoms such as depression, or the like. There have been developed various neuropharmacological reward therapies using representative drugs including such as Madopar as a levodopa (L-dopa) ingredient which is a dopamine precursor, bromidine and lisuride as a dopamine receptor agonist ingredient, artane as an anti-acetylcholline drug, and cogentin. Among them, levodopa, which is a precursor of dopamine, has been most effectively used to improve symptoms of Parkin's disease by supplementing the concentration of deficient dopamine in the brain, but the administration of levodopa during a long-term period of 3 to 5 years or more results in adverse effects such as a wearing-off phenomenon in which drug effective time is gradually shortened, an on-off phenomenon in which the fluctuation of motion control function against the effect of drug becomes serious, and an abnormal motion symptom (diskinesia) (Freed et. al., N. Engl. J. Med. 327:1549-55(1992)).

In addition, as a surgical therapy for Parkinson's disease, thalamotomy, pallidotomy, deep brain stimulation, neuronal cell transplantation, and the like have been performed. However, lasting time of therapeutic effect differs significantly from patient to patient, and infrequently, surgical therapy is accompanied by side effects such as hypophonia caused by surgery, dysarthria, and a decline in memory (Ondo et. al., Neurology 50:266-270 (1998); Shannon et. al., Neurology 50:434-438(1998)).

The recent treatment direction for Alzheimer's disease, which is one of the side effects such as a decline in memory has been emphasized on the possibility that Alzheimer's disease results from cholinergic signaling and transmission whose functions are damaged in the cerebral cortex and the hippocampus (Bartus et al., Science. 217(4558): 408-14(1982)); and Coyle et al., Science. 219(4589):1184-90(1983)). Because this region in the brain is associated with memory and intelligence, functional defect in this part of the brain may cause definite damage to memory and judgment and loss of intellectual ability. Although the exact process of damage to neuronal signaling is still controversial, sensory plaque and neurofibrillary tangle (NFT) are considered as main causes of nerve damage. Sensory plaque due to the accumulation of amyloid β (Aβ) is the biggest feature of this disease, and Alzheimer's disease can be confirmed by a postmortem examination (Khachaturian, Arch. Neurol. 42(11):1097-105(1985)).

In the case of Alzheimer's disease, drugs and treatment methods for increasing an amount of acetylcholine to inhibit the impairment of cholinergic signaling or causing acetylcholine to be present for a long period of time, or causing acetylcholine to act more effectively on transmission of neuronal cells have been suggested, and thus, many compounds which increase the activity of acetylcholine of patients with Alzheimer's disease have been used. Currently, the most effective approach is a method of inhibiting the activity of acetylcholinesterase which blocks the neuronal signaling by rapidly decomposing acetylcholine in the synapse. Actually, these inhibitors (for example: tacrine, donepezil, and rivastigmine) are currently on the market as therapeutic drugs for Alzheimer's disease, which are approved by the FDA. In many cases, the drugs are effective in preventing the destructive progression of the disease, but are not well applied to the return of the nervous system to a pre-illness state.

Although the development of treatment methods to reduce the effects of Alzheimer's disease has been actively underway, the current method is to provide a temporary improvement in symptoms. In conclusion, the treatment of Alzheimer's disease currently focuses on improving the symptoms of the disease rather than reversing the progression of the disease. The biological knowledge of the disease has been known widely, but the clinical application results have not yet been successful.

Meanwhile, stroke is a cerebrovascular disease that causes cerebral blood vessels to rupture or become clogged, resulting in abnormal functioning of local brain tissue, is often referred to as apoplexy, and is the leading cause of death in Korea. Furthermore, due to the extension of life expectancy caused by industrialization and medical development, the incidence thereof tends to increase gradually. Stroke may occur in any part of the brain, and thus, may impair almost every function of the body. Medically, a stroke may be classified into 'ischemic stroke' that appears because the blood vessels in the brain are clogged and blood cannot be circulated at specific sites and 'hemorrhagic stroke' caused by cerebral hemorrhage, and between them, the incidence ratio of ischemic stroke that is closely related to hypertension and arteriosclerosis which are known to be responsible for adult disease is higher than that of hemorrhagic stroke.

Ischemic stroke can occur when blood vessels are clogged, everywhere, from the carotid artery in the neck part, the vertebrobasilar artery to the fine arteries in the brain, and as a result, a phenomenon in which the brain tissue governed by the blood vessel dies, that is, 'cerebral infarction' occurs. Once the cerebral infarction site has occurred, the function thereof cannot be restored, and thus the central nervous system disorder due to cerebral infarction is not recovered and remains permanent. Therefore, the most important thing in the treatment of stroke is prevention against cerebral ischemia itself in addition to prevention methods of hypertension, diabetes, hyperlipidemia, and the like known to be risk factors of the occurrence of stroke. Further, when cerebral infarction occurs due to the onset of stroke, a focus is made on the reduction of secondary brain damage by reducing brain edema and allowing a portion in an ischemic state to be appropriately circulated.

Examples of substances currently used for neuronal protection are excitatory amino acid antagonists such as ganglioside and nimodipine, and GABA agonists such as clomethiazole, magnesium sulfate and glycine antagonist are under Phase II clinical trial, and a large-scale clinical trial is being currently performed about piracetam. However, the neuroprotective agents currently attempted are preparations acting on different steps in ischemia development process, and thus there still remains a need for developing a complex therapy acting simultaneously on these various processes, but there still remains a need for solving side effects and drug interaction problems.

Further, since symptoms of ischemic stroke are abruptly developed without specific prognosis, the uptake of a functional food for constantly preventing ischemia and inhibiting post-ischemic neuronal apoptosis has been determined to be more effective than treatment expected through drugs administered at the time of onset of cerebral ischemia.

Currently in the clinical setting, the cognitive function related diseases are treated in combination with pharmacotherapy and long-term psychotherapy, and in the case of drug treatment, benzodiazepine-based anti-anxiety drugs as diazepam, lorazepam, clonazepam, and alprazolam are usually used, and azapirone-based buspirone is used as a drug that may selectively act on serotonin receptors to selectively relieve anxiety symptoms. Further, recently, studies on stress-regulating substances derived from natural products which may compensate for side effects of these drugs have been actively conducted, and thus, there is a great need for foods with improved cognitive function, which have the function of actually treating stress anxiety and depression.

### [Prior Art Document]

### [Patent Document]

(Patent Document 1) KR 10-1787432 B1
(Patent Document 2) Korean KR/ Patent Laid-Open Official Gazette A/ Registered Official Gazette B1

### [Disclosure]

### [Technical Problem]

An object of the present invention is to provide a material effective for improving cognitive function, including a Clematis terniflora var. mandshurica extract as an active ingredient.

Another object of the present invention is to provide a pharmaceutical composition for improving cognitive function by using the Clematis terniflora var. mandshurica extract which is a natural extract as an active ingredient.

Still another object of the present invention is to provide a functional food composition for improving cognitive function by using the Clematis terniflora var. mandshurica extract which is a natural extract as an active ingredient.

### [Technical Solution]

To achieve the objects, the composition for improving cognitive function, including the Clematis terniflora var. mandshurica extract according to an exemplary embodiment of the present invention as an active ingredient includes the Clematis terniflora var. mandshurica extract as a first natural extract, and includes a second natural extract selected from the group consisting of a dandelion extract, a monocotyledon extract, an Indigofera pseudotinctoria Matsum extract, a Hydrangea serrata extract, a castanea crenata shell extract, a plantain extract, a Ligularia fischeri extract, a Ligularia stenocephala extract, and a mixture thereof.

The first natural extract and the second natural extract may be extracted with a solvent selected from the group consisting of water, an alcohol having 1 to 10 carbon atoms, and a mixed solvent thereof.

The composition may further include a Capsicum annuum extract.

The cognitive function relates to o learning ability, memory ability, or concentration.

The composition for improving cognitive function may improve the deterioration of brain or cognitive function accompanied by a cerebral nervous disease.

The present invention relates to a pharmaceutical composition including the composition for improving cognitive function.

The present invention relates to a functional food composition including the composition for improving cognitive function.

Hereinafter, the present invention will be described in more detail.

In the present invention, "natural extract" means an active ingredient isolated from natural products.

In the present specification, "nerve cells" include neurons, nerve supporting cells, glia, Schumann cells, and the like which form a structure such as the central nervous system, brain, brain stem, spinal cord, and the junction between the central nervous system and the peripheral nervous system.

In the present specification, "protection of nerve cells" means an action of reducing or ameliorating a nervous insult or an action of protecting or restoring nerve cells damaged by the nervous insult.

In the present specification, "nervous insult" means any damage to nerve cells or nerve tissues caused by various causes (for example: metabolic causes, toxic causes, neurotoxic causes and chemical causes, and the like).

In the present specification, a neurological disease means any disorder caused by the above-described damage to nerve cells or nerve tissues, and in particular, a cerebral nerve disease means any disorder caused by damage to nerve cells or nerve tissues in the brain.

In the present specification, "prevention" means the inhibition of the occurrence of a disorder or disease in an animal that has never been diagnosed as having a disorder or disease, but is prone to the disorder or disease.

In the present specification, "treatment" means (a) inhibition of development of a disorder or disease; (b) reduction of the disorder or disease; and (c) elimination of the disorder or disease.

The composition for improving cognitive function, including the Clematis terniflora var. mandshurica extract according to an exemplary embodiment of the present invention as an active ingredient may include the Clematis terniflora var. mandshurica extract as a first natural extract, and may include a second natural extract selected from the group consisting of a dandelion extract, a monocotyledon extract, an Indigofera pseudotinctoria Matsum extract, a Hydrangea serrata extract, a castanea crenata shell extract, a plantain extract, a Ligularia fischeri extract, a Ligularia stenocephala extract, and a mixture thereof.

Clematis terniflora var. mandshurica is a deciduous vine plant that grows in many mountains and fields in Korea. As a growth environment, Clematis terniflora var. mandshurica grows in places with high soil fertility in the sun or in the semi-shade. The height is 2 to 4 m, the leaves are facing each other and the leaflets are oval, but the apex is gradually narrow, and the base is rounded or wedged. The petiole is bent, and thus is the same as a tendril, has no hair on both sides, and has a flat tip. The flowers are white and about 1.2 to 2 cm in length, and bloom at the end of the stems and on the axilla of the leaves. The fruits ripen around September. The fruits are used for ornamental purposes, young leaves are used for food, and the roots are used for medicinal purposes.

Dandelion (Taraxacum platycarpum Dahlst) is a perennial herb that grows well in many mountains and fields in Korea. As a growth environment, dandelion grows regardless of soil fertility in the semi-shade or in the sun. The flowers are yellow and 3 to 7 cm in diameter, and hang on flower stems as long as the leaves. The fruits are black seeds and have silver egrets attached thereto. The difference between the dandelion and the western dandelion (Taraxacum officinale Weber) can be seen from the receptacle, and the native dandelion in Korea still has the receptacle, but the western dandelion is stretched down. This is the easiest method to distinguish. Young leaves are used for food, and whole plants including roots are used for medicinal purposes.

Monocotyledon (Lepisorus thunbergianus (Kaulf.) Ching) is an evergreen perennial and propagates by rhizomes and spores. Monocotyledon is distributed in Jeju and the southern region, and grows on the surface of moist rocks or the trunk of old trees. The rhizomes that extend sideways are about 2 to 3 mm in diameter, and there are narrow needle-shaped scales on the surface. The leaves seem to sprout in groups because the leaves come out from rhizomes where the distance between gnarls is short, are linear and about 10 to 20 cm in length, and the tips are pointed. The sorus is round, hangs in a row on both sides of the upper midrib on the back side, and is yellowish. There is no theca. Unlike 'Lepisorus onoei (Franch. & Sav.) Ching', the rhizome is thick and about 2 to 3 mm in diameter, and the leaves are 10 to 30 cm in length and about 5 to 15 mm in width. Lepisorus thunbergianus (Kaulf.) Ching is also used for ornamental and bonsai purposes, and is used for medicinal purposes.

Indigofera pseudotinctoria Matsum is a deciduous broad-leaved shrub and grows sideways by cutting many branches, and the twigs have sericeous hairs and are thin. The height is about 2 m. The leaves have 5 to 11 small leaves, are oval-shaped obovate, oval, or long oval, and have a petiole of 1 to 3 cm odd pinnate compound leaf. The edge is flat, the tip of the leaf is dull or concave, or the bottom of the leaf is round. The root sites are used for medicinal purposes.

Hydrangea serrata is a Hydrangea plant that belongs to the family Hydrangeaceae and consists of approximately 23 species of woody shrubs. The western hemisphere and easern Asia are the origins thereof. Examples of the Hydrangea plant growing in Korea include H. serrata, Hydrangea petiolaris, and the like. H. serrata and H. petiolaris grow in mountains and fields, and the types to be planted in gardens are Hydrangea macrophylla and varieties thereof. H. serrata can be found all over Korea, but H. petiolaris grows only in Ulleungdo Island and Jeju Island. In addition, Hydrangea paniculata, Hydrangea arborescens, and the like are imported from foreign countries and planted in gardens and the like for landscaping purposes.

The chestnut tree (Castanea crenata Sieb) is a deciduous broad-leaved shrub belonging to the family Fagaceae, and its fruit chestnut (Castane crenata) is composed of fruit, inner skin, and outer skin. The inner skin also called castanea crenata shell tastes bitter, and thus is generally used after being removed to use chestnut contents for food, but a castanea crenata shell extract is used for medicinal purposes.

Ligularia stenocephala grows in the wetlands of deep mountains. The height is 60 to 100 cm. The whole plant has no hair and the root is thick. The leaves on the roots remain until flowers bloom, but are 24 cm long and 20 cm wide. Hair grows along the veins on the back side of the leaves, and there are sharp saw blades on the edges. The petiole is wide about 40 cm long with no wings, and the base is wide. There are three leaves that hang to the stem, and the lower ones are almost the same as the leaves on the roots, but the higher the position is, the shorter the petiole is and the petiole becomes a leaf sheath, and the smaller the leaves are. The flowers are yellow, and arranged in a racemose inflorescence at the end of the stem from August to September. The flowers bloom from the bottom to the top, and are about 3 cm in diameter. The bract is lancet-shaped and 2 to 3 mm in length, and the flower stalk is 1 to 3.5 cm in length. The total bract has a narrow barrel shape, and there are 5 bract pieces. The corolla is 20 to 25 mm long and 3 to 4 mm wide. The pappus is shorter and lighter brown than the corolla. The fruit is an achene, ripens in October, and is upside-down lancet-shaped. The color is brownish-white, and is 6 to 7 mm long.

Plantain contains plantenolic acid, adenine, pholin, and the like, and has better efficacy than ginseng or deer antler, and has anti-cancer effects. Since leaf stems of plantain have a substance that act as a diuretic, plantain dried in the sun is decocted and drunk for chronic constipation. Fresh leaves may be eaten as green juice, and may be squashed, put into hot water, and drunk instead of tea. Since the whole plants or seeds have an ingredient to remove phlegm, 15 to 20 g of the whole plants or seeds are decocted daily and drunk between meals. Even when edema occurs from nephritis, salt absorption is reduced and 5 to 15 g of seeds are decocted daily and drunk. Seeds are effective for relief of edema and for diuresis, stethocatharsis, antibacterial, antiinflammatory, anti-cancer, jaundice, tonsils, hemostasis, and furunculosis.

Ligularia fischeri is a perennial grass belonging to the family Asteraceae. Ligularia fischeri is found in deep mountains. Ligularia fischeri is about 1 m tall, and the root leaf has a long leaf plug and is shaped like a heart, Ligularia fischeri has sharp serrated teeth, the stem leaves are small, and the lower parts wrap stems in a sheath. In July to September, yellowish flowers bloom in a racemose inflorescence.

When the Clematis terniflora var. mandshurica extract is used alone, it is possible to exhibit excellent effects for improving cognitive function.

However, compared to the case where the Clematis terniflora var. mandshurica extract is used alone, when a second natural extract is mixed and used as a composite extract, a synergistic effect is exhibited by the effect of the mixture between the composite natural extracts.

That is, compared to the case where the Clematis terniflora var. mandshurica extract is used alone, when the Clematis terniflora var. mandshurica extract is used in mixture with those selected from the group including a second natural extract selected from the group consisting of a dandelion extract, a monocotyledon extract, an Indigofera pseudotinctoria Matsum extract, a Hydrangea serrata extract, a castanea crenata shell extract , a plantain extract, a Ligularia fischeri extract, a Ligularia stenocephala extract, and a mixture thereof are mixed and used, the effect of improving cognitive function is further enhanced by the composition of the composite extract.

Further, the Clematis terniflora var. mandshurica extract has a better effect by using a fraction obtained by fractionating an extract extracted from dried Clematis terniflora var. mandshurica. Preferably, the Clematis terniflora var. mandshurica extract is a Clematis terniflora var. mandshurica methyl chloride fraction.

Specifically, a natural extract may be obtained by including: grinding a dried natural product; leaching the ground product by using an organic solvent; leaching a sample, and then drying the sample; re-leaching the dried sample by using an organic solvent; leaching the sample, and then drying the sample; leaching the sample by using water; and leaching the resulting product.

The natural product may be selected from the group consisting of Clematis terniflora var. mandshurica, dandelion, monocotyledon, Indigofera pseudotinctoria Matsum, Hydrangea serrata, castanea crenata shell, plantain, Ligularia fischeri, and Ligularia stenocephala.

The method may further include performing fractionation on the natural extract extracted by using the organic solvent by using an organic solvent.

The method for preparing the extract may be a typical extraction method in the art, such as an ultrasonic extraction method, a leaching method, and a reflux extraction method. Specifically, a natural product from which impurities are removed by washing and drying may be an extract extracted with water, an alcohol having 1 to 6 carbon atoms, or a mixed solvent thereof, and may be an extract extracted by sequentially applying the solvents to the sample.

The reflux extraction method is performed by 10 to 30 g of a ground material of the natural product based on 100 mL of an alcohol having 1 to 6 carbon atoms, a reflux time of 1 to 3 hours, and a 50 to 100% alcohol having 1 to 6 carbon atoms. More specifically, the method is performed by 10 to 20 g of a ground material of the natural product based on 100 mL of an alcohol having 1 to 6 carbon atoms, a reflux time of 1 to 2 hours, and a 70 to 90% alcohol having 1 to 4 carbon atoms.

The leaching method is performed by a 50 to 100% alcohol having 1 to 6 carbon atoms at 15 to 30°C for 24 to 72 hours. More specifically, the leaching method is performed by a 70 to 80% alcohol having 1 to 6 carbon atoms at 20 to 25°C for 30 to 54 hours.

The ultrasonic extraction method is performed by a 50 to 100% alcohol having 1 to 6 carbon atoms at 30 to 50°C for 0.5 to 2.5 hours. Specifically, the ultrasonic extraction method is performed by a 70 to 80% alcohol having 1 to 6 carbon atoms at 40 to 50°C for 1 to 2.5 hours.

The extraction solvent may be used 2 to 50 times, more specifically 2 to 20 times, based on the weight of the sample. For extraction, the sample may be left to stand in the extraction solvent for leaching for 1 to 72 hours, more specifically, 24 to 48 hours.

After extraction, the extract may be fractionated by sequentially applying new fractionation solvents. For a fractionation solvent used during the fractionation, the solvent is one or more selected from the group consisting of water, hexane, butanol, ethyl acetic acid, ethyl acetate, methylene chloride, and a mixture thereof, preferably, ethyl acetate or methylene chloride.

After the extract or fraction is obtained, a method such as concentration or lyophilization may be additionally used.

The composition for improving cognitive function of the present invention may additionally include a Capsicum annuum extract.

More preferably, the Capsicum annuum extract is a red hot pepper fruit stalk extract.

The red hot pepper fruit stalk extract is extracted by using the same method as the method for preparing a natural extract after separating a stalk part from a red hot pepper fruit, drying the stalk part, and pulverizing the stalk part.

Compared to the case where red hot pepper pulp or the whole red hot pepper is used, when an extract is extracted from a red hot pepper fruit stalk, and then used as an ingredient of a composition for improving cognitive function, the action of improving cognitive function may be doubled by an active ingredient included in the red hot pepper fruit stalk.

Thus, preferably, the composition for improving cognitive function, including the Clematis terniflora var. mandshurica extract of the present invention as an active ingredient includes the Clematis terniflora var. mandshurica extract as a first natural extract, includes a second natural extract selected from the group consisting of a dandelion extract, a monocotyledon extract, an Indigofera pseudotinctoria Matsum extract, a Hydrangea serrata extract, a castanea crenata shell extract, a plantain extract, a Ligularia fischeri extract, a Ligularia stenocephala extract, and a mixture thereof, and may additionally include a red hot pepper fruit stalk extract.

More preferably, the composition for improving cognitive function may additionally include, based on 100 parts by weight of the Clematis terniflora var. mandshurica extract, 50 to 100 parts by weight of the dandelion extract; 40 to 60 parts by weight of the monocotyledon extract; 40 to 60 parts by weight of the Indigofera pseudotinctoria Matsum extract; 40 to 60 parts by weight of the Hydrangea serrata extract; 40 to 60 parts by weight of the castanea crenata shell extract; 40 to 60 parts by weight of the plantain extract; 40 to 60 parts by weight of the Ligularia fischeri extract; 50 to 80 parts by weight of the Ligularia stenocephala extract; and 30 to 50 parts by weight of the red hot pepper fruit stalk extract. When the natural extract is used within the above range, the effect of improving cognitive function may be further enhanced by the mixing action between the respective constituent ingredients.

Specific examples of a disease to which the composition of the present invention may be applied include a neurodegenerative disease, a disease caused by ischemia or refusion, a mental disease, and the like, but are not limited thereto. More preferably, the composition of the present invention may be used for the prevention or treatment of a neurodegenerative disease such as dementia, Alzheimer's disease, Huntington's disease, Parkinson's disease, and amyotrophic lateral sclerosis; a disease caused by damage to nerve cells due to ischemia such as stroke (particularly ischemic stroke) or refusion; and (c) a mental disease such as schizophrenia, depression, and post-traumatic stress disorder.

The composition including the natural composite extract according to an exemplary embodiment of the present invention is particularly useful for the prevention or treatment of a disease (for example: stroke) caused by damage to nerve cells due to ischemia or refusion.

The aforementioned efficacy is usually manifested by the nerve cell protecting action of the natural composite composition of the invention. The nerve cell protecting action by the composition including the natural composite extract may be exerted through various mechanisms, for example, by suppressing the death of nerve cells, and examples of the death of these nerve cells include necrosis and apoptosis of nerve cells. When the apoptosis of nerve cells is inhibited, one of the targets of the composition including the natural composite extract of the present invention is caspase (Guy et.al., Cell 91:443-446(1987)), which inhibits the apoptosis by inhibiting the activity of the enzyme.

The composition including the natural composite extract also exhibits a very excellent effect in enhancing cognitive function. The composition including the natural composite extract of the present invention exhibits all the excellent efficacies in various indices in support for enhancing cognitive function, for example, memory quotient (MQ), learning slope, memory retentiveness, recall efficiency, drawing/memory consensus, language/view consensus, intelligence/memory consensus, short term memory, and attentive concentration, and thus generally acts on greatly enhancing the cognitive function of humans.

Further, the composition including the natural composite extract according to an exemplary embodiment of the present invention exhibits an excellent effect for improving or preventing the deterioration of the cognitive function accompanied by the aforementioned neurological disease.

In particular, the composition including the natural composite extract enhances cognitive function by inhibiting a decrease in the amount of acetylcholine in the brain.

In addition, the composition including the natural composite extract prevents damage to cognitive function by inhibiting the death of nerve cells caused by a stroke.

According to an exemplary embodiment of the present invention, the improvement of cognitive function exhibited by the composition including the natural composite extract of the present invention is an enhancement of learning ability and/or memory ability.

While having been known to have excellent effects for the treatment and prevention of dementia by increasing the concentration of acetylcholine to improve cognitive ability and block the progression of dementia, a choline agonist or a cholinesterase inhibitor has been used for patients. Examples of currently developed drugs include Lecithin as an acetylcholine precursor, RS-86 and nicotine as a receptor agonist, and the like, Tacrine as an acetylcholinesterase inhibitor approved by the FDA and commercially available even in Korea, Aricept recently approved, and the like, but these drugs are still in a controversial state for use because the effects thereof are temporary and weak and the drugs are seriously toxic.

However, since the composition including the natural composite extract of the present invention has extremely low toxicity to the human body, the composition is very useful as a medicine or functional food for enhancing cognitive function.

A pharmaceutically acceptable carrier included in the pharmaceutical composition of the present invention is that typically used in formulation, and includes carbohydrate compounds (for example: lactose, amylose, dextrose, sucrose, sorbitol, mannitol, starch, cellulose, and the like), gum acacia, calcium phosphate, alginate, gelatin, calcium silicate, microcrystalline cellulose, polyvinylpyrrolidone, cellulose, water, a syrup, a salt solution, an alcohol, gum arabic, polyethylene glycol, methyl cellulose, methyl hydroxybenzoate, propyl hydroxybenzoate, talc, magnesium stearate, mineral oil, and the like, but is not limited thereto. The pharmaceutical composition of the present invention may additionally include a lubricant, a wetting agent, a sweetening agent, a flavoring agent, an emulsifier, a suspending agent, a preservative, and the like, in addition to the aforementioned ingredients, but is not limited thereto.

The pharmaceutical composition of the present invention may be administered orally or parenterally, and in the case of parenteral administration, the composition may be administered by intravenous injection, subcutaneous injection, intramuscular injection or the like.

A suitable administration amount of the pharmaceutical composition of the present invention may vary depending on factors, such as formulation method, administration method, age, body weight, gender or disease condition of a patient, diet, administration time, administration route, excretion rate and response sensitivity, and a doctor with ordinary skill may readily determine and prescribe an administration amount effective for a desired therapy or prophylaxis. According to a preferred exemplary embodiment of the present invention, the suitable administration amount is 50 mg to 10 g once a day on an adult basis.

The pharmaceutical composition of the present invention may be prepared in the form of a unit-dose or by being contained in a multi-dose container by being formulated using a pharmaceutically acceptable carrier and/or excipient according to a method that can be readily implemented by a person with ordinary skill in the art to which the present invention pertains. In this case, a dosage form may also be in the form of a solution in an oil or aqueous medium, a suspension or in the form of an emulsion, an extract, a powder, a granule, a tablet or a capsule, and the pharmaceutical composition of the present invention may additionally include a dispersant or a stabilizer.

Meanwhile, a functional food composition of the present invention includes an ingredient typically added during the preparation of food, and include, for example, proteins, carbohydrates, fats, nutrients, and seasonings. For example, when the functional food composition of the present invention is prepared as a drink, the composition may additionally include citric acid, liquid fructose, sugar, sucrose, acetic acid, malic acid, a fruit juice, a legume extract, a jujube extract, a licorice extract, and the like in addition to the silk peptide and natural extract of the present invention. In consideration of a ready approach to food, the food of the present invention is very useful for treating or preventing a neurological disease, treating or preventing a disease caused by oxidative stress, and improving cognitive function.

The composition of the present invention not only exhibits various effects as described above, but also includes a natural product as an active ingredient, and therefore has extremely fewer side effects on the human body than chemically synthesized drugs.

### [Advantageous Effects]

According to the composition for improving cognitive function, including the Clematis terniflora var. mandshurica extract of the present invention as an active ingredient, it is possible to provide a composition effective for improving cognitive function by utilizing an extract derived from natural products.

The composition for improving cognitive function, including the Clematis terniflora var. mandshurica extract of the present invention as an active ingredient can be provided as a pharmaceutical composition to provide a composition with enhanced effect of improving cognitive function without any side effects.

Further, the functional food composition of the present invention can also be provided as a functional food composition with high preference while exhibiting effects of improving cognitive function.

### [Description of Drawings]

FIG. 1 relates to a graph illustrating effects of the mixtures of the natural composite extracts of the present invention on the cerebral infarction site caused by ischemia.
FIG. 2 relates to a graph illustrating anti-depressant effects when the mixtures of the natural composite extracts of the present invention are acutely treated once.
FIG. 3 relates to a graph illustrating anti-depressant effects when the mixtures of the natural composite extracts of the present invention are repeatedly treated for a long period of time.
FIG. 4 relates to a graph illustrating amounts of cortisol in blood when the mixtures of the natural composite extracts of the present invention are repeatedly treated for a long period of time.
FIG. 5 is a graph for experiments of measuring dopamine of the natural composite extract of the present invention.
FIG. 6 is a graph for experiments of measuring serotonin of the natural composite extract of the present invention.

### [Best Mode]

Hereinafter, the Examples of the present invention will be described in detail such that a person skilled in the art to which the present invention pertains can easily carry out the present invention. However, the present invention can be implemented in various different forms, and is not limited to the Examples described herein.

### [Preparation Example 1: Preparation of natural extract]

### 1. Preparation of Clematis terniflora var. mandshurica (E1) extract

### 1) Extraction of sample

After dried Clematis terniflora var. mandshurica was leached at room temperature for 48 hours by using 70% ethanol, a sample was filtered and concentrated.

### 2) Acquisition of fraction (E2)

After 1 g of the Clematis terniflora var. mandshurica extract extracted with 70% ethanol was dissolved in 30 ml of distilled water, fractionation was performed three times using hexane, and the separated aqueous layer was again fractionated three times. In the three-time fractionation step, the Clematis terniflora var. mandshurica extract was fractionated by using methyl chloride. After the fractionation three times, the separated aqueous layer was again fractionated three times by using ethyl acetate, the separated aqueous layer was again fractionated three times by using n-butanol, and each fraction was secured by concentrating the obtained solution.

### 2. Preparation of natural extract

A concentrate was prepared from dandelion (E3), monocotyledon (E4) Indigofera pseudotinctoria Matsum (E5), Hydrangea serrata (E6), castanea crenata shell (E7), plantain (E8), Ligularia fischeri (E9), Ligularia stenocephala (E10), red hot pepper fruit (E11), and red hot pepper fruit stalk (E12) by using the same method as in that of the Clematis terniflora var. mandshurica extract. Thereafter, a natural extract was prepared by dissolving 1 g of each concentrate in 30 ml of distilled water.

### [Preparation Example 2: Preparation of composition for improving cognitive function]

According to the composition as in the following Table 1, compositions for improving cognitive function of S1 to S7 were prepared by mixing the constituent ingredients.

**[Table 1]**

| | S1 | S2 | S3 | S4 | S5 | S6 | S7 |
|---|---|---|---|---|---|---|---|
| E1 | 100 | 100 | 100 | 100 | - | - | - |
| E2 | - | - | - | - | 100 | 100 | 100 |
| E3 | 40 | 50 | 80 | 120 | 50 | 100 | 100 |
| E4 | 30 | 40 | 50 | 80 | 40 | 60 | 60 |
| E5 | 30 | 40 | 50 | 80 | 40 | 60 | 60 |
| E6 | 30 | 40 | 50 | 80 | 40 | 60 | 60 |
| E7 | 30 | 40 | 50 | 80 | 40 | 60 | 60 |
| E8 | 30 | 40 | 50 | 80 | 40 | 60 | 60 |
| E9 | 30 | 40 | 50 | 80 | 40 | 60 | 60 |
| E10 | 30 | 40 | 50 | 80 | 40 | 60 | 60 |
| E11 | - | - | - | - | - | - | 40 |
| E12 | 20 | 30 | 40 | 60 | 30 | 40 | - |

| | | | | | | | |
|---|---|---|---|---|---|---|---|
| (Unit parts by weight) | | | | | | | |

### [Experimental Example 1: Experiment of ischemic animal model]

In order to confirm the effects of the compositions including the natural extract prepared in the Preparation Examples on the area of cerebral infarction caused by ischemia, the following experiment was performed.

### Experimental Example 1-1: Local ischemia-induced animal model

### A. Treatment with drug and construction of local ischemia-induced animal model

A local ischemia-induced animal model was constructed using a male white rat (Sprague-Dawley rat) weighing 200 to 250 g. As a drug, 1 g/kg of each of Experimental Nos. S1 to S7 was orally administered once 1 hour before the induction of ischemia or once 1 hour after the induction of ischemia (oral administration group, n = 6). After the experimental animal was anesthetized by injecting 30 to 40 mg/kg of ketamine intramuscularly to the animal, the skin of the neck was incised in the supine position, and the common carotid artery, the external carotid artery, and the internal carotid artery were located and separated from surrounding tissues. First, the superior parathyroid gland artery and the posterior fossa artery, which are branches of the external carotid artery, and the pterygopalatine artery, which is a branch of the internal carotid artery, were electrocauterized, and the external carotid artery were cut. Occlusion of origin of middle cerebral artery was performed by incorporating 4-0 nylon thread (ETHICON, INC) into the internal carotid artery through the external carotid artery, and then placing 16 to 18 mm of nylon thread within the branch of the common carotid artery.

### B. Experiments of effects of ischemia on reduction of cerebral infarction site

Brains were removed by decapitating the animal 6 hours after induction of ischemia. The removed brains were cut at an interval of 2 mm from anterior pole, and reacted with a 2% triphenyl tetrazolium chloride (TTC, Sigma) solution at 37°C for 30 minutes, followed by fixing in a 4% paraformaldehyde (Sigma) solution. After stained tissue sections were photographed, the area of a region that turned white due to the occurrence of cerebral infarction was measured unlike the red-stained normal region by using MCID image processing system (Imaging Research Inc.) for the photographs, the ratio of the cerebral infarction area to the area of the entire brain section was obtained, and then the average value was calculated. Further, the ratio of the volume occupied by the cerebral infarction site in the entire volume from each brain section was measured.

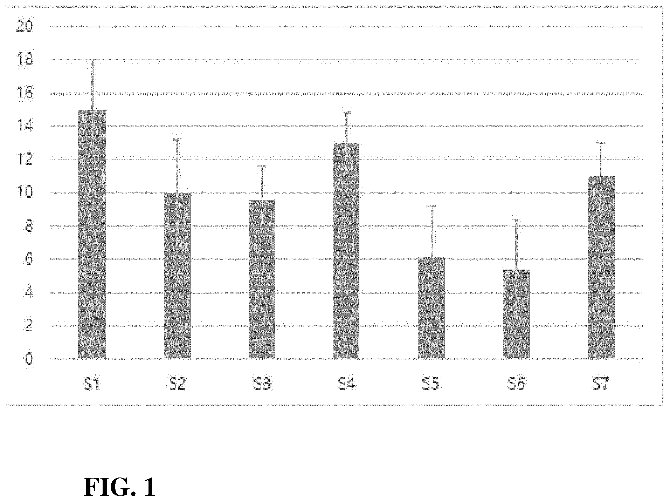

The composition including the natural composite extract of the present invention is reducing the cerebral infarction site caused by ischemia to a level significant to the control group. According to FIG. 1, as a result of measuring the ratio of the volume of the cerebral infarction to the volume of the entire brain section, pretreatment of S2, S3, S5, and S6 showed a significant effect on the reduction of cerebral infarction site caused by local ischemia.

In particular, it was confirmed that S5 and S6 showed beneficial results. In addition, through the experiment, it was confirmed that the case where the red hot pepper fruit stalk was used was excellent in effect as compared to the case where the red hot pepper fruit was used (S7). In the accompanying FIG. 1, each measured value shows the mean ± standard deviation.

### [Experimental Example 2: Experiment of depression animal model]

### Experimental Example 2-1: Preparation of experimental animals

Experimental animals were male white rats (Sprague Dawley, 140 to 180 g, Korea Experimental Animal Center), and these animals were adapted to the environment by placing four animals per breeding box for a week in a predetermined environment (indoor temperature 25±1°C, relative humidity 60±10%) and allowing the animals to uptake water and feed without limitation, and then used for the next experiment. Among the white rats, the animals which showed little movement and a deteriorated developmental situation in a general breeding state and an abnormal stereotyped behavior or markedly reduced swimming ability in forced swimming were excluded, and 50 animals in total were used for the present experiment.

### Experimental Example 2-2: Experiment of depression animal model

In the present experiment, a forced swimming test (FST), which is a standardized test method also called a behavioral despair test, was used. Ecologically, white rats dislike water, and thus, when exposed to water, will exhibit characteristics behaviors to get out of the environment. That is, experimental animals will forcibly swim to get out of water and will no longer swim after a certain period of time, and by using this principle, the forced swimming test method is known as a basic experiment which will search for antidepressant effects during the drug development. The FST process was performed as follows according to the method of Porsolt et al., who were the first proposers (Porsolt et al., Eur. J. Pharmacol. 51(3), 291-294, 1978).

First, a transparent acrylic cylindrical water tank with a height of 40 cm and a diameter of 18 cm was filled with water at 25°C to a height of 15 cm, and the white rats in Experimental Example 2-1 were forced to swim the water tank, and then left for 15 minutes. Although the rat violently tried to go out of the tank for the first several minutes, the immobilization time increased as time went by, and the rat maintained its body in the almost immobilization state for the last several minutes. The typical immobilization state refers to a state in which a white rat floats on the water with only a part of its face on the surface of the water, with only a slight movement to keep the body balanced. After the forced swimming, the white rat body's moisture was wiped off, the body was dried with a 37 ° C dryer for 30 minutes, and the animal was returned to the breeding box.

24 hours after the first forced swimming, the second forced swimming was performed. In the present experiment, for the second forced swimming, the white rats were allowed to stay in the water bath for only 5 minutes under the same condition as the first forced swimming test, and the total immobilization time during this period was measured. During the second forced swimming, as a result of learned helplessness, most of the white rats show more immobilization time than the first forced swimming. Since the increase in immobilization state, which is regarded as a symptom indicator of depression in the forced swimming model, is reduced again by the treatment with the antidepressant agents, the decrease in immobilization state is considered to be related to the action of the antidepressant agents. Since some drugs do not show the effect during an acute treatment with the drug, but show the effect during a long-term treatment in some cases in the forced swimming test, in the present experiment, treatment with the drug was performed for 7 days, and then the second forced swimming was attempted. This method is often used in forced swimming tests because the antidepressant effect of antidepressants is generally obtained after drug administration for at least 2 weeks. The immobilization state was measured by video recording the entire process during the second forced swimming, and the immobilization times between the control group and the experimental group were compared and evaluated by measuring the immobilization time. Through video screen analysis, three trained evaluators obtained an average value among the evaluators by counting the immobilization time with a second clock, and used the average value as an analysis data.

### Administration method

Experimental Nos. S1 to S7 of the present invention were prepared as 100 mg/ml solutions, and thus orally administered. All the samples were dissolved in distilled water and administered orally, and administered 1 hour before the second forced swimming at the time of single administration, and when administered repeatedly for a long period of time for 7 days, administered 30 minutes after the first forced swimming, and then repeatedly administered for 7 days.

### Antidepressant effect during acute treatment once

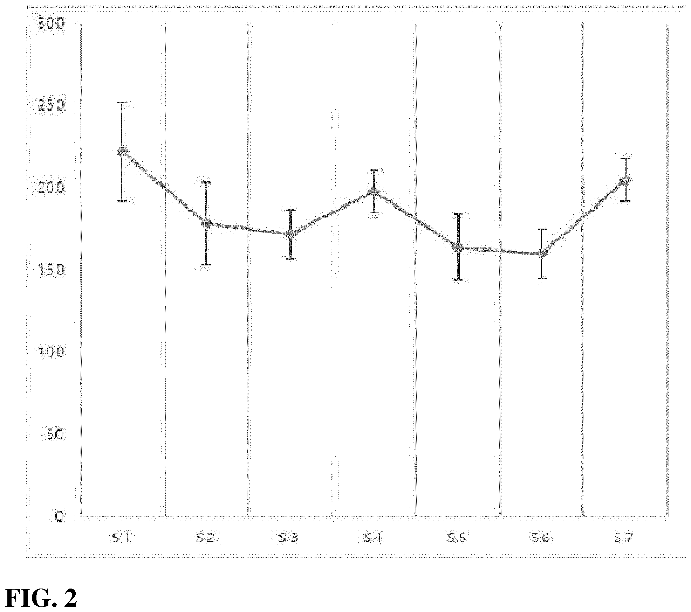

During the acute treatment with S1 to S7 of the present invention once (1 g/kg), the antidepressant effect was confirmed. The administration amount was set at 20 mg/kg during administration once with reference to the results of existing studies (Eur. J. Pharmacol. 138(3), 413-416, 1987; Neuropharmacology 28(3), 229-233, 1989). The experimental results are illustrated in FIG. 2.

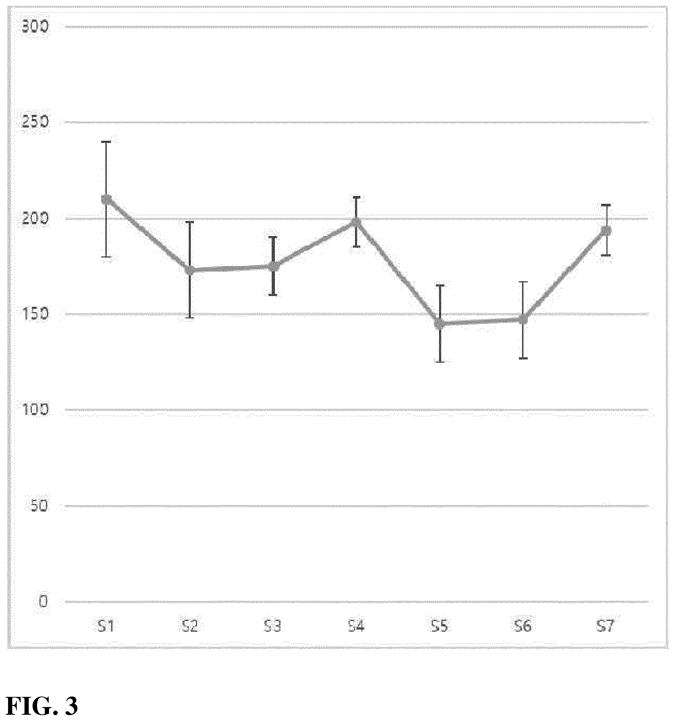

As illustrated in FIG. 2, it can be seen that the average immobilization time caused by the acute treatment is significantly reduced in the experimental group to which S2, S3, S5, and S6 were administered (**, P < 0.05). In the accompanying FIG. 3, each measured value shows the mean ± standard deviation.

### Antidepressant effect during long-term repeated treatment (for 7 days)

During the long-term repeated treatment with S1 to S7 of the present invention, the antidepressant effect was confirmed. The same experiment as the single treatment was performed by orally administering S1 to S7 at 50 mg/kg once daily for 7 days. The experimental results are illustrated in FIG. 3.

As illustrated in FIG. 3, the average immobilization time caused by the long-term treatment was reduced by administration of S2, S3, S5, and S6 (P<0.1). In the accompanying FIG. 3, each measured value shows the mean ± standard deviation.

### [Experimental Example 3: Effects of preventing and improving stress]

### Experimental Example 3-1: Preparation of experimental animals

4-week-old ICR male mice {SAMTAKO Bio Korea, Korea} were subjected to an adaptation time of 1 week, and then bred in a space where lighting is turned on and off repeatedly in a unit of 12 hours, and an indoor temperature of 18 to 23°C and a humidity of 60% were maintained. As a feed, a solid feed was supplied, and there was no restriction on the feed or grade other than the process of inducing stress.

### Experimental Example 3-2: Stress induction

The stress-inducing method is used by modifying the method of Wilner et al. (Reduction of sucrose preference by chronic unpredictable mild stress, 1987), and specifically, stress is induced by creating a variety of unexpected mentally stressful situations such as fasting, dietary restriction after fasting (feeding a small amount of feed), suspension of water supply, provision of empty jugs after suspension of water supply, tilted breeding farm, breeding of a number of experimental animals in a breeding farm, sparkling light, cold room, and continuous light.

### 1. Comparison of amount of cortisol in blood

The amounts of cortisol in blood were measured by administering the compositions for improving cognitive function of S1 to S7 to the experimental animals prepared above.

### Administration Method

Experimental Nos. S1 to S7 of the present invention were orally administered at 50 mg/kg once daily for 7 days. All the samples were dissolved in distilled water and orally administered, and repeatedly administered for a long term for 7 days.

### Amount of cortisol in blood during long-term repeated treatment (for 7 days)

During the long-term repeated treatment with S1 to S7 of the present invention, the amount of cortisol in blood was confirmed.

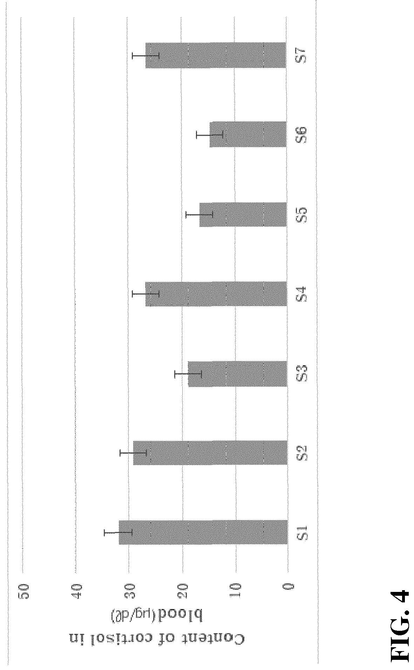

For the experimental results, as illustrated in FIG. 4, it can be seen that the average cortisol amount is significantly decreased in the experimental group to which S2, S3, S5, and S6 are administered.
Each measured value exhibits the average ± standard deviation.

### 2. Effects of preventing inhibition of dopamine

Dopamine is a precursor of norepinephrine as a neurotransmitter in the central nervous system, and is involved in cognition and attention concentration, reward, regulation of motor function, and the like. Since it is known that dopamine secretion is inhibited in a chronic stress situation, the stress prevention and improvement effects caused by the composition of the present invention were confirmed through a dopamine measurement experiment.

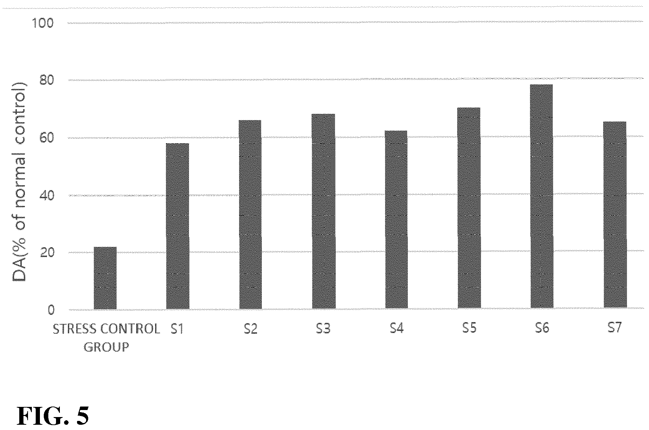

As a result of measuring the dopamine concentration using the high performance liquid chromatography with electrochemical detection (HPLC-ECD) method, the value (% of control) compared with the dopamine concentration of the normal control group was shown as an average and standard deviation. The administration groups of the compositions for improving cognitive function of S1 to S7 and the stress control group were compared by verifying the significance with a statistical analysis using a Student's T-test, and p<0.05 was considered to be significant and displayed. The results are illustrated in FIG. 5.

Referring to FIG. 5, it can be seen that the concentration of dopamine measured from the hippocampus of the test animals subjected to stress is remarkably lower than that in the normal control group. By the way, it can be seen that the concentration of dopamine is maintained at higher levels in the groups to which the compositions for improving cognitive function of S1 to S7 are administered than in the stress control group.

In particular, it may mean that in the experimental groups to which S2, S3, S5, and S6 are administered, the effect of inhibiting reduction of dopamine is excellent.

### 3. Effect of improving serotonin

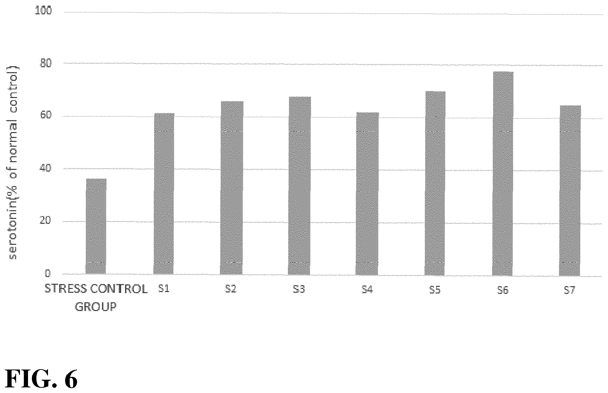

As a result, the value (% of control) compared with the serotonin concentration of the normal control group was expressed as the mean and standard deviation. The administration groups of the compositions for improving cognitive function of S1 to S7 and the stress control group were compared by verifying the significance with a statistical analysis using a Student's T-test, and p<0.05 was considered to be significant and displayed. The results are illustrated in FIG. 6.

Referring to FIG. 6, it can be seen that the concentration of serotonin measured from the hippocampus of the test animals subjected to stress is remarkably lower than that in the normal control group. By the way, it can be seen that the concentration of serotonin was maintained at higher levels in the groups to which the compositions for improving cognitive function of S1 to S7 than in the stress control group.

In particular, it may mean that in the experimental groups to which S2, S3, S5, and S6 were administered, the effect of inhibiting reduction of serotonin is excellent.

Therefore, in the case of the above range, it can be seen that the effect of relieving stress is shown by inhibiting the reduction of serotonin caused by stress.

### [Experimental Example 4: Preference test]

### 1. Sensory evaluation test

Tea beverages were prepared by diluting the compositions for improving cognitive function of S1 to S7. The tea beverages were sampled by 10 tasters, and the taste and aroma were expressed by an index of 1 to 10, and the average value (applied to a rounding of 0.05) is shown in the following Table 2. The higher the number of the index is, the higher the preference is.

**[Table 2]**

| | S1 | S2 | S3 | S4 | S5 | S6 | S7 |
|---|---|---|---|---|---|---|---|
| Taste | 6.0 | 6.5 | 6.5 | 6.0 | 7.0 | 7.5 | 6.0 |
| Flavor | 6.0 | 6.5 | 6.5 | 6.5 | 7.5 | 7.5 | 6.5 |
| Overall preference (average) | 6.0 | 6.5 | 6.5 | 6.3 | 7.3 | 7.5 | 6.3 |

| | | | | | | | |
|---|---|---|---|---|---|---|---|
| (Unit: Index) | | | | | | | |

Referring to Table 2, it can be seen that in the case of the compositions of improving the cognitive function of S2, S3, S5, and S6, the preference is enhanced. Therefore, in the case of using the compositions for improving cognitive function of S2, S3, S5, and S6, it is possible to provide a functional food for improving cognitive function, which is excellent in stress relief and antidepressant effects with flavor and taste of higher preference.

Although preferred Examples of the present invention have been described in detail hereinabove, the right scope of the present invention is not limited thereto, and it should be understood that many variations and modifications of those skilled in the art using the basic concept of the present invention, which is defined in the following claims, will also fall within the right scope of the present invention.

## Claims

1. A composition for improving cognitive function, comprising a Clematis terniflora var. mandshurica extract as an active ingredient, the composition comprising the Clematis terniflora var. mandshurica extract as a first natural extract, and
a second natural extract selected from the group consisting of a dandelion extract, a monocotyledon extract, an Indigofera pseudotinctoria Matsum extract, a Hydrangea serrata extract, a castanea crenata shell extract, a plantain extract, a Ligularia fischeri extract, a Ligularia stenocephala extract, and a mixture thereof.

2. The composition of claim 1, wherein the first natural extract and the second natural extract are extracted with a solvent selected from the group consisting of water, an alcohol having 1 to 10 carbon atoms, and a mixed solvent thereof.

3. The composition of claim 1, further comprising a Capsicum annuum extract.

4. The composition of claim 1, wherein the cognitive function is learning ability, memory ability, or concentration.

5. The composition of claim 1, wherein the composition improves the deterioration of brain or cognitive function accompanied by a cerebral nervous disease.

6. The composition of claim 1, wherein the composition exhibits stress relief, mood improvement and antidepressant effects.

7. A pharmaceutical composition for improving cognitive function, comprising the composition according to any one of claims 1 to 6.

8. A functional food composition for improving cognitive function, comprising the composition according to any one of claims 1 to 6.
